# EUROPEAN PATENT APPLICATION

(11) **EP 4 273 212 A1**
(43) Date of publication of application: **08.11.2023**
(21) Application number: 21914194.2
(22) Date of filing: 24.12.2021
(51) Int. Cl.: C12M 1/00, C12M 1/34

(54) **AUTOMATIC LOADING SYSTEM FOR EXTRACTION STRIP**

(30) Priority: 29.12.2020 CN 202011604641
(71) Applicant: Autobio Labtec Instruments Co., Ltd., Zhengzhou, Henan 450016 (CN)
(72) Inventor: ZHAO, Peng, Zhengzhou, Henan 450016 (CN); PAN, Yang, Zhengzhou, Henan 450016 (CN); LIU, Yaoji, Zhengzhou, Henan 450016 (CN); WANG, Chao, Zhengzhou, Henan 450016 (CN); LIU, Cong, Zhengzhou, Henan 450016 (CN)
(74) Representative: Kolster Oy Ab
(86) International application number: PCT/CN2021/141194
(87) International publication number: WO 2022/143457

(57) **Abstract**

An automatic loading system for an extraction strip (3), the automatic loading system comprising: an extraction strip (3) for holding several reagents, a first channel (1), a second channel (2) parallel to and attached to the first channel (1), a first hand pushing device (4) for pushing the extraction strip (3) to move in the first channel (1), a second hand pushing device (5) for pushing the extraction strip (3) to move in the second channel (2), and a transfer device (6) for transferring the extraction strip (3) from the first channel (1) to the second channel (2), wherein one end of the first channel (1) is a loading inlet of the extraction strip (3); one end of the second channel (2) is a grabbing position of the extraction strip (3); the transfer device (6) is arranged at the other ends of the first channel (1) and the second channel (2); and the first hand pushing device (4) and the second hand pushing device (5) can move transversely and move up and down. The on-line adding operation of the extraction strip (3) can be achieved, such that there is no need to immediately interrupt the previous pushing and transport operation every time a new extraction strip (3) is added, which is beneficial for improving the degree of automation and use effect of the device. In addition, the automatic loading system has a simple structure, is convenient use, and can be popularized and used.

## Description

This application claims the benefit of the priority to Chinese Patent Application No. 202011604641.X, titled "AUTOMATIC LOADING SYSTEM FOR EXTRACTION STRIP", filed with the China National Intellectual Property Administration on December 29, 2020, which is incorporated herein by reference in its entirety.

### FIELD

The present application relates to the technical field of extraction strips, and in particular to an automatic loading system for an extraction strip.

### BACKGROUND

At present, for most of the nucleic acid extraction systems on the market, samples are fed in batches, but most of the processes such as supply of samples, supply of extraction reagent strips and discard of consumables are carried out manually, which can no longer meet the market demand of projects with a large number of diagnosis samples.

In the conventional technology, a few automatic loading systems for the extraction strip mainly include a test box, a loading passage, a conveying passage and a box pusher. The loading passage is used to receive the test box and transmit the test box to the conveying passage. The conveying passage is used to convey the test box into the automatic system for tests. But the existing box pusher can only move left and right. Therefore, each time a new test box is added, the box pusher needs to be returned to an initial position to then push the test box for conveying.

To sum up, how to improve the automation degree and use effect of the loading system for the extraction strip is a problem to be urgently solved by those skilled in the art.

### SUMMARY

In view of the above, an object of the present application is to provide an automatic loading system for an extraction strip, which can effectively improve the automation degree and use effect of the loading system for the extraction strip. In addition, the device is simple in structure and convenient in use, and can be popularized.

In order to achieve the above object, the following technical solutions are provided according to the present application.

An automatic loading system for an extraction strip includes an extraction strip configured to hold multiple reagents, a first passage, a second passage which is parallel and adjacent to the first passage, a first pushing device configured to push the extraction strip to move in the first passage, a second pushing device configured to push the extraction strip to move in the second passage, and a transfer device configured to transfer the extraction strip from the first passage to the second passage, wherein one end of the first passage is a loading inlet for the extraction strip, one end of the second passage is a grabbing position for the extraction strip, and the transfer device is arranged at another end of the first passage and another end of the second passage; and both the first pushing device and the second pushing device are configured to move left and right and to move up and down.

Preferably, the automatic loading system for the extraction strip further includes a frame, wherein a length of the first passage is the same as a length of the second passage, and the frame is arranged to surround an outer periphery of the first passage and an outer periphery of the second passage.

Preferably, the transfer device is arranged on a right side of the frame and is configured to move forward and backward along the right side of the frame.

Preferably, the first passage and the second passage each are provided with a sliding groove for the extraction strip to slide transversely.

Preferably, a right end of the first passage is provided with a separation position, a first sensor configured to detect whether the extraction strip reaches the separation position, and a first back pushing device configured to push back the extraction strip; and
a left end of the second passage is provided with a second sensor configured to detect whether the extraction strip reaches the grabbing position, and a second back pushing device configured to push back the extraction strip.

Preferably, the left end of the second passage is provided with a jacking device configured to move up and down to separate the extraction strip.

Preferably, the jacking device includes a pushing plate configured to extract the extraction strip, and a first lifting device configured to drive the pushing plate to move up and down, wherein the pushing plate is horizontally arranged, and the first lifting device is a rack-and-pinion mechanism.

Preferably, the first pushing device and the second pushing device each include a pushing member configured to move up and down to push the extraction strip to move, and a transverse movement assembly configured to drive the pushing member to move transversely, wherein the pushing member is slidably arranged on the transverse movement assembly, and the transverse movement assembly is a synchronous belt mechanism.

Preferably, the pushing member includes a left pusher, a right pusher configured to be used in cooperation with the left pusher, and a second lifting device configured to drive the left pusher and the right pusher to synchronously move up or down, wherein the left pusher and the right pusher are vertically arranged, and the second lifting device is a rack-and-pinion mechanism.

Preferably, an avoidance hole for the left pusher and the right pusher, which have been lowered, to pass is defined in the extraction strip.

When the automatic loading system for the extraction strip according to the present application is used, first, the extraction strip is loaded at the loading inlet of the first passage, and then, the extraction strip is pushed to move in the first passage when the first pushing device moves transversely. When the extraction strip reaches the right end of the first passage, the extraction strip can be transferred to the second passage by the transfer device, and then the extraction strip is conveyed from the right end to the left end of the second passage by the second pushing device. When the extraction strip reaches the grabbing position of the second passage, other grabbing device can grab the extraction strip, so as to facilitate other operations on the extraction strip.

The movement, conveying and storage of the extraction strip can be achieved in the first passage and the second passage. In addition to the transverse movement, the first pushing device and the second pushing device of the device can also move up and down. Therefore, when a new extraction strip is added in the first passage or the second passage, the first pushing device or the second pushing device can continue pushing the extraction strip transversely, without returning to the initial position immediately. After the operation of adding the extraction strip is completed, the first pushing device or the second pushing device is controlled to lower first to avoid collision with the extraction strip, and then the first pushing device or the second pushing device is moved to the initial position. When the newly added extraction strip needs to be pushed again, the first pushing device or the second pushing device is controlled to rise to push the newly added extraction strip again. Therefore, the device can achieve the on-line adding of the extraction strip, such that there is no need to immediately interrupt the on-going pushing and conveying every time a new extraction strip is added, which is beneficial to improving the automation degree and use effect of the device.

In summary, the automatic loading system for the extraction strip according to the present application can effectively improve the automation degree and use effect of the automatic loading system for the extraction strip. In addition, the device is simple in structure and convenient in use, and can be popularized.

### BRIEF DESCRIPTION OF THE DRAWINGS

For more clearly illustrating the technical solutions in the embodiments of the present application or in the conventional technology, drawings referred to for describing the embodiments or the conventional technology will be briefly described hereinafter. Apparently, the drawings in the following description are only some embodiments of the present application, and for the person skilled in the art, other drawings may be obtained based on the provided drawings without any creative efforts.
FIG. 1 is a schematic structural view of an automatic loading system for an extraction strip according to the present application;
FIG. 2 is a schematic structural view of a first pushing device and a second pushing device;
FIG. 3 is a schematic structural view of a pushing member;
FIG. 4 is a schematic structural view of a jacking device;
FIG. 5 is a schematic view showing a lowered left pusher and a lowered right pusher for passing through an avoidance hole; and
FIG. 6 is a schematic view showing a state where the extraction strip is separated by the jacking device.

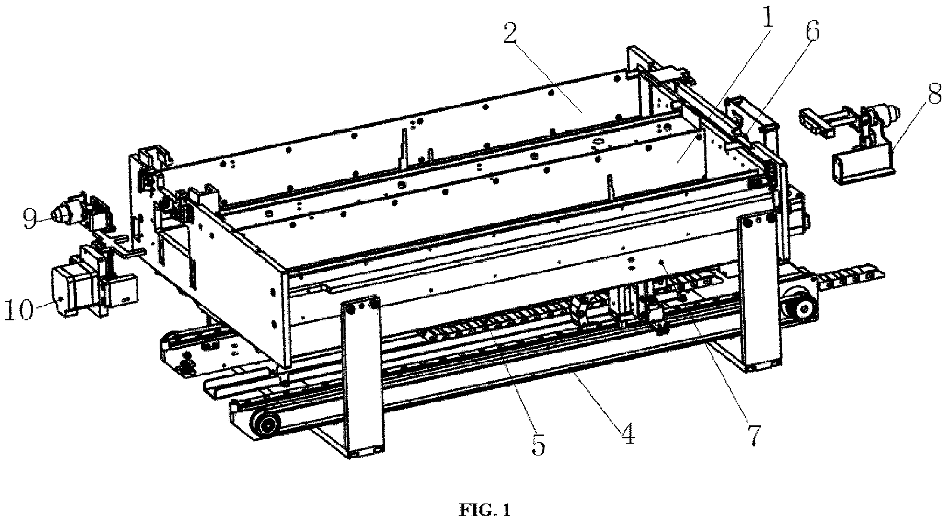

Reference numerals in FIGS. 1 to 6 are listed as follows:
1 first passage, 2 second passage, 3 extraction strip, 31 avoidance hole, 4 first pushing device, 5 second pushing device, 6 transfer device, 7 frame, 8 first back pushing device, 9 second back pushing device, 10 jacking device, 101 connecting plate, 102 stepping motor, 103 motor fixing plate, 104 first spur gear, 105 first toothed rack, 106 first linear guide rail, 107 first sliding plate, 108 pushing plate, 11 pushing member, 111 left pusher, 112 right pusher, 113 outer mounting plate, 114 lifting motor, 115 second spur gear, 116 second toothed rack, 117 pusher fixing plate, 118 outer linear rail seat, 119 second linear guide rail, 1110 second sliding plate, 12 transverse movement assembly, 121 outer linear rail plate, 122 synchronous motor, 123 motor mounting plate, 124 synchronous belt pulley, 125 idler pulley, 126 synchronous belt, 127 cantilever pin, 128 straight guide rail.

### DETAILED DESCRIPTION OF THE EMBODIMENTS

Technical solutions in the embodiments of the present application will be described clearly and completely hereinafter in conjunction with the drawings in the embodiments of the present application. Apparently, the embodiments described in the following are only some embodiments of the present application, rather than all embodiments. Any other embodiments obtained by those skilled in the art on the basis of the embodiments of the present application without any creative work fall within the scope of protection of the present application.

A core of the present application is to provide an automatic loading system for an extraction strip, which can effectively improve the automation degree and use effect of the loading system for the extraction strip. In addition, the device is simple in structure and convenient in use, and can be popularized.

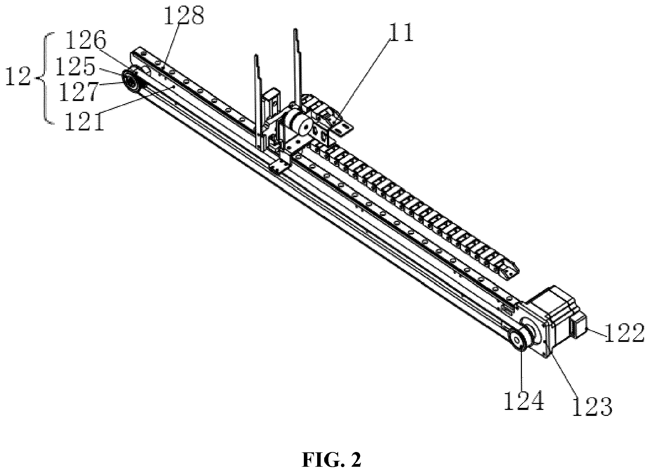

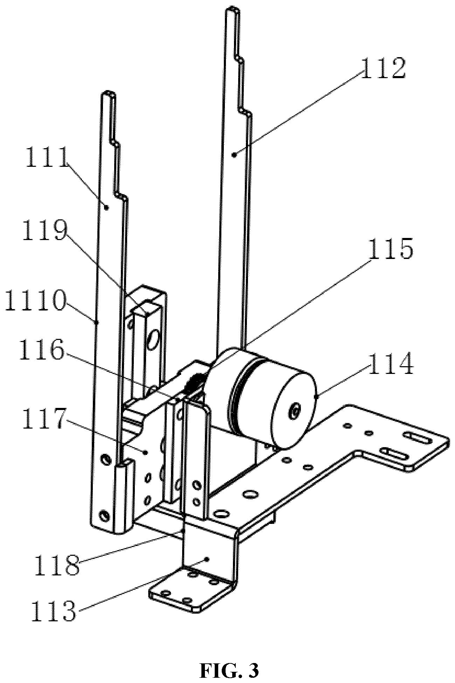

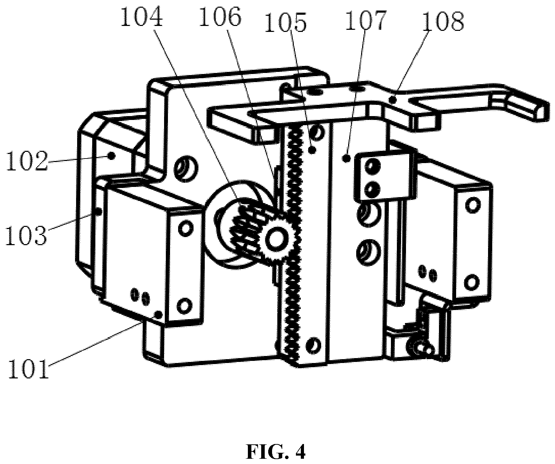

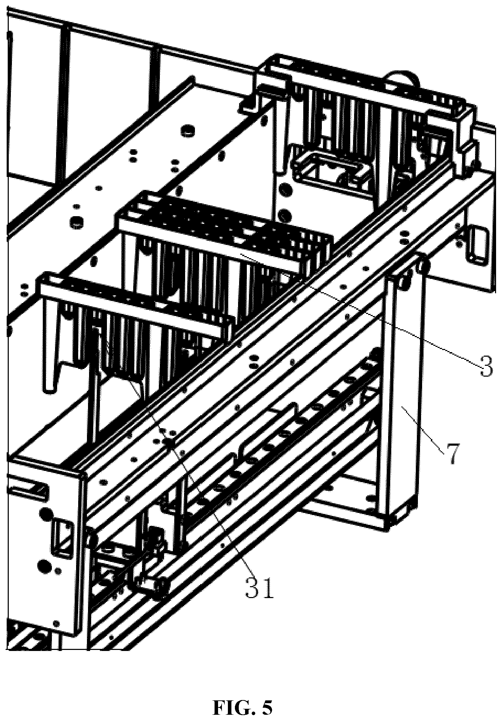

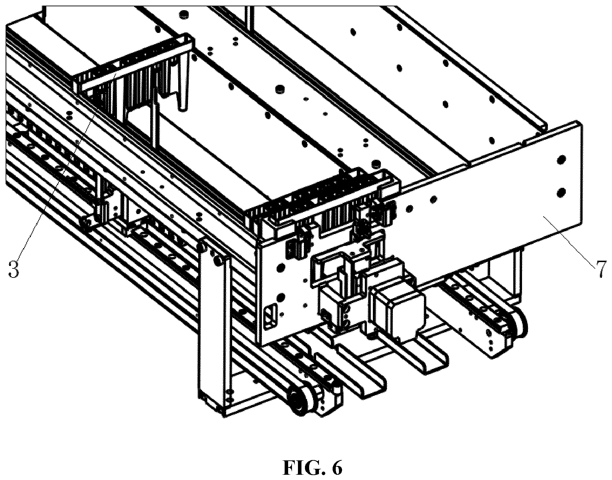

Referring to FIG. 1 to FIG. 6, FIG. 1 is a schematic structural view of an automatic loading system for an extraction strip according to the present application; FIG. 2 is a schematic structural view of a first pushing device and a second pushing device; FIG. 3 is a schematic structural view of a pushing member; FIG. 4 is a schematic structural view of a jacking device; FIG. 5 is a schematic view showing a lowered left pusher and a lowered right pusher for passing through an avoidance hole; and FIG. 6 is a schematic view showing a state where the extraction strip is separated by the jacking device.

An automatic loading system for an extraction strip is provided according to a specific embodiment, which includes an extraction strip 3 configured to hold multiple reagents, a first passage 1, a second passage 2 which is parallel and adjacent to the first passage 1, a first pushing device 4 configured to push the extraction strip 3 to move in the first passage 1, a second pushing device 5 configured to push the extraction strip 3 to move in the second passage 2, and a transfer device 6 configured to transfer the extraction strip 3 from the first passage 1 to the second passage 2. One end of the first passage 1 is a loading inlet for the extraction strip 3, one end of the second passage 2 is a grabbing position for the extraction strip 3, and the transfer device 6 is arranged at another end of the first passage 1 and another end of the second passage 2. Both the first pushing device 4 and the second pushing device 5 are configured to move left and right and to move up and down.

For example, a left end of the first passage 1 is arranged as the loading inlet for the extraction strip 3, a left end of the second passage 2 is arranged as the grabbing position for the extraction strip 3, and the transfer device 6 is arranged at a right end of the first passage 1 and a right end of the second passage 2.

It should be noted that both the first pushing device 4 and the second pushing device 5 are configured to move left and right and to move up and down, so that the device has the function of on-line adding the extraction strip 3. That is, for the newly added extraction strip 3, the first pushing device 4 and the second pushing device 5 are controlled to lower, then the first pushing device 4 and the second pushing device 5 are controlled to move horizontally to a rear side of the newly added extraction strip 3, and then the first pushing device 4 and the second pushing device 5 are controlled to rise and continue to push the extraction strip 3 horizontally forward, until the extraction strip 3 reaches a preset position.

In the actual application process, shapes, structures, sizes, positions, and the like of the extraction strip 3, the first passage 1, the second passage 2, the first pushing device 4, the second pushing device 5 and the transfer device 6 may be determined according to the actual situation and actual needs.

When the automatic loading system for the extraction strip according to the present application is used, first, the extraction strip 3 is loaded at the loading inlet of the first passage 1, and then, the extraction strip 3 is pushed to move in the first passage 1 when the first pushing device 4 moves transversely. When the extraction strip 3 reaches the right end of the first passage 1, the extraction strip 3 can be transferred to the second passage 2 by the transfer device 6, and then the extraction strip 3 is conveyed from the right end to the left end of the second passage 2 by the second pushing device 5. When the extraction strip 3 reaches the grabbing position of the second passage 2, other grabbing device can grab the extraction strip 3, so as to facilitate other operations on the extraction strip 3.

The movement, conveying and storage of the extraction strip 3 can be achieved in the first passage 1 and the second passage 2. In addition to the transverse movement, the first pushing device 4 and the second pushing device 5 of the device can also move up and down. Therefore, when a new extraction strip 3 is added in the first passage 1 or the second passage 2, the first pushing device 4 or the second pushing device 5 can continue pushing the extraction strip 3 transversely, without returning to the initial position immediately. After the operation of adding the extraction strip 3 is completed, the first pushing device 4 or the second pushing device 5 is controlled to lower first to avoid collision with the extraction strip 3, and then the first pushing device 4 or the second pushing device 5 is moved to the initial position. When the newly added extraction strip 3 needs to be pushed again, the first pushing device 4 or the second pushing device 5 is controlled to rise to push the newly added extraction strip 3 again. Therefore, the device can achieve the on-line adding of the extraction strip 3, such that there is no need to immediately interrupt the on-going pushing and conveying every time a new extraction strip 3 is added, which is beneficial to improving the automation degree and use effect of the device.

In summary, the automatic loading system for the extraction strip according to the present application can effectively improve the automation degree and use effect of the automatic loading system for the extraction strip 3. In addition, the device is simple in structure and convenient in use, and can be popularized.

On the basis of the above embodiments, preferably, the automatic loading system for the extraction strip further includes a frame 7. A length of the first passage 1 is the same as a length of the second passage 2. The frame 7 is arranged to surround an outer periphery of the first passage 1 and an outer periphery of the second passage 2. The frame 7 is a mounting base for various parts of the device, so that the overall structure of the device is more compact.

Preferably, the transfer device 6 is arranged on a right side of the frame 7 and is configured to move forward and backward along the right side of the frame 7.

It should be noted that, when the extraction strip 3 moves to the right end of the first passage 1, the transfer device 6 is controlled to drive the extraction strip 3 to move backward, so as to move the extraction strip 3 to the right end of the second passage 2. Then, the transfer device 6 releases the extraction strip 3, so that the second pushing device 5 pushes the extraction strip 3 to move in the second passage 2. At the same time, the transfer device 6 moves forward to the initial position, so as to facilitate the transferring operation of the next extraction strip 3.

Preferably, the first passage 1 and the second passage 2 each are provided with a sliding groove for the extraction strip 3 to slide transversely. Therefore, when the first pushing device 4 and the second pushing device 5 push the extraction strip 3, the extraction strip 3 can slide smoothly in the first passage 1 and the second passage 2.

In the actual application process, shapes, structures, sizes, materials, and the like of the frame 7 and the sliding grooves may be determined according to the actual situation and actual needs.

On the basis of the above embodiments, preferably, the right end of the first passage 1 is provided with a separation position, a first sensor configured to detect whether the extraction strip 3 reaches the separation position, and a first back pushing device 8 configured to push back the extraction strip 3; the left end of the second passage 2 is provided with a second sensor configured to detect whether the extraction strip 3 reaches the grabbing position, and a second back pushing device 9 configured to push back the extraction strip 3.

It should be noted that, when the extraction strip 3 moves to the separation position of the first passage 1, the extraction strip 3 is transferred to the second passage 2 by the transfer device 6. When the extraction strip 3 moves to the grabbing position of the second passage 2, other grabbing device grabs the extraction strip 3, so as to facilitate other detection operations.

It should be further noted that, the first pushing back device 8 and the second pushing back device 9 do not need to be used when the device is in normal operation to convey the extraction strips 3. However, when the device needs to be restarted or initialized in case of failure, the first pushing back device 8 and the second pushing back device 9 need to be used to push the extraction strip 3 located at the separation position or the grabbing position back into the first passage 1 or the second passage 2, so as to avoid disturbing the normal operation of the device.

In the actual application process, positions, shapes, sizes, structures and the like of the first sensor, the second sensor, the first pushing back device 8 and the second pushing back device 9 may be determined according to the actual situation and actual needs.

Preferably, the left end of the second passage 2 is provided with a jacking device 10 configured to move up and down to separate the extraction strip 3.

It should be noted that, the jacking device 10 is provided at the grabbing position so as to separate multiple huddled extraction strips 3. The extraction strip 3 which is jacked up by the jacking device 10 can be more conveniently and accurately grabbed by other grabbing structures.

In addition, it should be noted that, when the automatic loading system for the extraction strip according to the present application is used, the extraction strip 3 is loaded into the first passage 1, and is pushed to move in the first passage 1 by the first pushing device 4. When the extraction strip 3 is moved to the separation position, the extraction strip 3 is transferred to the second passage 2 by the transfer device 6, and then the extraction strip 3 is pushed to move in the second passage 2 by the second pushing device 5. When the extraction strip 3 reaches the grabbing position, the extraction strip 3 is jacked by the jacking device 10 to separate, so that other grabbing structures can grab the extraction strip 3.

Preferably, the jacking device 10 includes a pushing plate 108 configured to extract a certain extraction strip 3, and a first lifting device configured to drive the pushing plate 108 to move up and down, wherein the pushing plate 108 is horizontally arranged, and the first lifting device is a rack-and-pinion mechanism.

It should be noted that, the first lifting device may include a connecting plate 101 fixedly connected with the frame 7, a stepping motor 102, a motor fixing plate 103 configured to mount the stepping motor 102, a first spur gear 104 sleeved on an outer circumference of an output shaft of the stepping motor 102, a first toothed rack 105 meshed with the first spur gear 104, a first linear guide rail 106 arranged on the motor fixing plate 103, and a first sliding plate 107 slidably connected with the first linear guide rail 106. The first toothed rack 105 is connected with the first sliding plate 107, and the pushing plate 108 is horizontally arranged at the top of the first sliding plate 107. When the stepping motor 102 rotates, it can drive the first spur gear 104 to rotate. The rotation of the first spur gear 104 drives the first toothed rack 105 to move up and down. The up-and-down movement of the first toothed rack 105 drives the first sliding plate 107 to move up and down on the first linear guide rail 106, and also drives the pushing plate 108 to move up and down, thus achieving the extraction and separation of the extraction strip 3.

It should be noted that, in addition to the rack-and-pinion mechanism, the first lifting device may also be embodied as other linear displacement mechanism. In the actual application process, shapes, structures, sizes, positions, and the like of the first lifting device and the pushing plate 108 may be determined according to the actual situation and actual needs.

On the basis of the above embodiments, preferably, the first pushing device 4 and the second pushing device 5 each include a pushing member 11 configured to move up and down to push the extraction strip 3 to move, and a transverse movement assembly 12 configured to drive the pushing member 11 to move transversely. The pushing member 11 is slidably arranged on the transverse movement assembly 12, and the transverse movement assembly 12 is a synchronous belt mechanism.

It should be noted that, the transverse movement assembly 12 may include an outer linear rail plate 121, a synchronous motor 122 arranged on one side of an end of the outer linear rail plate 121, a motor mounting plate 123 configured to fix and mount the synchronous motor 122, a synchronous belt pulley 124 sleeved on an outer circumference of an output shaft of the synchronous motor 122, an idler pulley 125 cooperating with the synchronous belt pulley 124, a synchronous belt 126 surrounding an outer circumference of the synchronous belt pulley 124 and an outer circumference of the idler pulley 125, a cantilever pin 127 configured to fix the outer linear rail plate 121 on the frame 7, and a straight guide rail 128 arranged at the top of the outer linear rail plate 121. The pushing member 11 is provided with an outer linear rail seat 118 which is slidably connected with the straight guide rail 128. Therefore, when the synchronous motor 122 rotates, it can drive the synchronous belt pulley 124 to rotate, further drive the idler pulley 125 to rotate by means of the synchronous belt 126, and further drive the pushing member 11 slidably mounted on the straight guide rail 128 to move linearly. Therefore, the pushing member 11 can push the extraction strip 3 to slide in the first passage 1 and the second passage 2. The pushing member 11 has the lifting function, which can achieve the on-line adding of the extraction strip 3.

Preferably, the pushing member 11 includes a left pusher 111, a right pusher 112 configured to be used in cooperation with the left pusher 111, and a second lifting device configured to drive the left pusher 111 and the right pusher 112 to synchronously move up or down. The left pusher 111 and the right pusher 112 are vertically arranged, and the second lifting device is a rack-and-pinion mechanism.

It should be noted that, the second lifting device may include an outer mounting plate 113 connected with the outer linear rail seat 118, a lifting motor 114 mounted above the outer mounting plate 113, a second spur gear 115 sleeved on an outer circumference of an outer shaft of the lifting motor 114, a second toothed rack 116 meshed with the second spur gear 115, a pusher fixing plate 117 configured to fix the left pusher 111 and the right pusher 112, a second linear guide rail 119 arranged on the outer linear rail seat 118, and a second sliding plate 1110 slidably connected with the second linear guide rail 119. The second toothed rack 116 and the second sliding plate 1110 are respectively arranged on a front side and a rear side of the pusher fixing plate 117. The left pusher 111 and the right pusher 112 are vertically arranged on a left side and a right side of the pusher fixing plate 117. Therefore, when the lifting motor 114 rotates, it can drive the second spur gear 115 to rotate. The rotation of the second spur gear 115 drives the second toothed rack 116 to move up and down, and the up-and-down movement of the second toothed rack 116 drives the second sliding plate 1110 to move up and down on the second linear guide rail 119, so as to drive the left pusher 111 and the right pusher 112 to synchronously move up or down.

Preferably, an avoidance hole 31 for the left pusher 111 and the right pusher 112, which have been lowered, to pass is defined in the extraction strip 3, so that the lowered left pusher 111 and the lowered right pusher 112 can smoothly avoid the extraction strip 3, so as to avoid hitting or knocking over the extraction strip 3.

It should be noted that, in addition to the rack-and-pinion mechanism, the second lifting device may also be embodied as other linear displacement mechanism. In the actual application process, shapes, structures, sizes, positions, and the like of the second lifting device, the left pusher 111, and the right pusher 112 may be determined according to the actual situation and actual needs.

It should be noted that, as used in the first lifting device, the second lifting device, the first passage 1, the second passage 2, the first pushing device 4, the second pushing device 5, the first sensor, the second sensor, the first pushing back device 8, the second pushing back device 9, the first sliding plate 107, the second sliding plate 1110, the first linear guide rail 106, the second linear guide rail 119, the first spur gear 104, the second spur gear 115, the first toothed rack 105 and the second toothed rack 116 mentioned herein, the terms "first" and "second" are only for distinguishing different positions, and do not imply a sequence in priority.

In addition, it should be further noted that the orientation or positional relationships indicated by the terms "left", "right", "front", "rear", "up", "down" and the like are based on illustrations in the drawings, and are merely for simplification of description and ease of understanding, rather than indicating or implying that the device or element referred to must have a particular orientation or be constructed and operated in the particular orientation, and therefore should not be construed as a limit to the present application.

The embodiments in this specification are described in a progressive manner. Each embodiment focuses on differences from other embodiments, and for the same or similar parts among the embodiments, reference can be made to one another. Any combination of all the embodiments provided in the present application is within the scope of protection of the present application, and will not be described herein.

The automatic loading system for the extraction strip according to the present application is described in detail hereinbefore. The principle and the embodiments of the present application are illustrated herein through specific examples. The description of the above embodiments is merely used to facilitate understanding the method and core idea of the present application. It should be noted that for those skilled in the art, various improvements and modifications can be made to the present application without departing from the principle of the present application, and these modifications and improvements are also deemed to fall into the scope of protection of the present application defined by the appended claims.

## Claims

1. An automatic loading system for an extraction strip, comprising: an extraction strip (3) configured to hold a plurality of reagents, a first passage (1), a second passage (2) which is parallel and adjacent to the first passage (1), a first pushing device (4) configured to push the extraction strip (3) to move in the first passage (1), a second pushing device (5) configured to push the extraction strip (3) to move in the second passage (2), and a transfer device (6) configured to transfer the extraction strip (3) from the first passage (1) to the second passage (2), wherein one end of the first passage (1) is a loading inlet for the extraction strip (3), one end of the second passage (2) is a grabbing position for the extraction strip (3), and the transfer device (6) is arranged at another end of the first passage (1) and another end of the second passage (2); and both the first pushing device (4) and the second pushing device (5) are configured to move left and right and to move up and down.

2. The automatic loading system for the extraction strip according to claim 1, further comprising a frame (7), wherein a length of the first passage (1) is the same as a length of the second passage (2); and the frame (7) is arranged to surround an outer periphery of the first passage (1) and an outer periphery of the second passage (2).

3. The automatic loading system for the extraction strip according to claim 2, wherein the transfer device (6) is arranged on a right side of the frame (7) and is configured to move forward and backward along the right side of the frame (7).

4. The automatic loading system for the extraction strip according to claim 1, wherein the first passage (1) and the second passage (2) each are provided with a sliding groove for the extraction strip (3) to slide transversely.

5. The automatic loading system for the extraction strip according to any one of claims 1 to 4, wherein a right end of the first passage (1) is provided with a separation position, a first sensor configured to detect whether the extraction strip (3) reaches the separation position, and a first back pushing device (8) configured to push back the extraction strip (3); and
a left end of the second passage (2) is provided with a second sensor configured to detect whether the extraction strip (3) reaches the grabbing position, and a second back pushing device (9) configured to push back the extraction strip (3).

6. The automatic loading system for the extraction strip according to any one of claims 1 to 4, wherein the left end of the second passage (2) is provided with a jacking device (10) configured to move up and down to separate the extraction strip (3).

7. The automatic loading system for the extraction strip according to claim 6, wherein the jacking device (10) comprises a pushing plate (108) configured to extract the extraction strip (3), and a first lifting device configured to drive the pushing plate (108) to move up and down, wherein the pushing plate (108) is horizontally arranged, and the first lifting device is a rack-and-pinion mechanism.

8. The automatic loading system for the extraction strip according to any one of claims 1 to 4, wherein the first pushing device (4) and the second pushing device (5) each comprise a pushing member (11) configured to move up and down to push the extraction strip (3) to move, and a transverse movement assembly (12) configured to drive the pushing member (11) to move transversely, wherein the pushing member (11) is slidably arranged on the transverse movement assembly (12), and the transverse movement assembly (12) is a synchronous belt mechanism.

9. The automatic loading system for the extraction strip according to claim 8, wherein the pushing member (11) comprises a left pusher (111), a right pusher (112) configured to be used in cooperation with the left pusher (111), and a second lifting device configured to drive the left pusher (111) and the right pusher (112) to synchronously move up or down, wherein the left pusher (111) and the right pusher (112) are vertically arranged, and the second lifting device is a rack-and-pinion mechanism.

10. The automatic loading system for the extraction strip according to claim 9, wherein an avoidance hole (31) for the left pusher (111) and the right pusher (112), which have been lowered, to pass is defined in the extraction strip (3).
